# EUROPEAN PATENT APPLICATION

(11) **EP 1 362 853 A1**
(43) Date of publication of application: **19.11.2003**
(21) Application number: 02710357.1
(22) Date of filing: 25.01.2002
(51) Int. Cl.: C07D 319/18, C07D 317/64, C07D 317/60, C07D 321/10, C07D 321/12, C07D 321/00, A61K 31/357

(54) **STYRENE DERIVATIVES AND PROCESS FOR PRODUCTION THEREOF**

(30) Priority: 26.01.2001 JP 2001017765
(71) Applicant: Kyowa Hakko Kogyo Co., Ltd, Tokyo 100-8185 (JP)
(72) Inventor: YANAGISAWA, Arata Sakai Research Laboratories, Sakai-shi, Osaka 590-8554 (JP); MOHRI, Shin-ichiro Sak. Pl. Kyowa H. Kog.Co. Ltd., Sakai-shi, Osaka 590-8554 (JP); YANASE, Masashi c/o Pharmaceutical Research Inst., DSunto-gun, Shizuoka 411-87 (JP)
(74) Representative: Casalonga, Axel
(86) International application number: JP0200554
(87) International publication number: WO02059105

(57) **Abstract**

The present invention provides a compound represented by general formula (I): 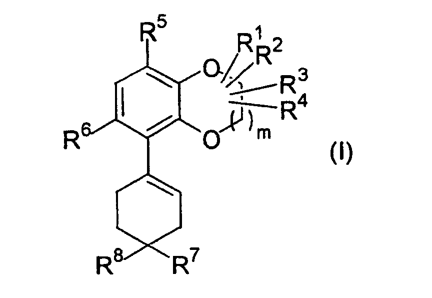 [wherein m represents an integer of 0 to 4; R¹, R², R³ and R⁴ may be the same or different and each represents a hydrogen atom, substituted or unsubstituted lower alkyl, or the like; R⁵ represents hydroxy, or substituted or unsubstituted lower alkoxy; R⁶ represents a hydrogen atom or halogen; R⁷ represents hydroxy, formyl, substituted or unsubstituted lower alkoxy, or the like; and R⁸ represents a hydrogen atom, substituted or unsubstituted lower alkoxy, or the like], or a salt thereof.

## Description

### Technical Field

The present invention relates to compound (I) described below that is useful as an intermediate for a preparation of a compound represented by general formula (XIII) described below [hereinafter the compound represented by general formula (XIII) may be referred to as compound (XIII). Compounds represented by other formula numbers may also be referred to in the same manner] which is useful as a phosphodiesterase-IV inhibitor (PDE-IV inhibitor).

### Background Art

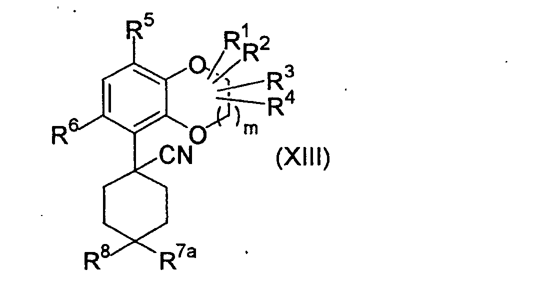

[In the formula, m represents an integer of 0 to 4, R¹, R², R³ and R⁴ may be the same or different and each represents a hydrogen atom, substituted or unsubstituted lower alkyl, substituted or unsubstituted cycloalkyl, polycycloalkyl, substituted or unsubstituted lower alkoxycarbonyl, substituted or unsubstituted lower alkanoyl, substituted or unsubstituted lower alkanoyloxy, cyano, hydroxy, substituted or unsubstituted lower alkoxy, substituted or unsubstituted cycloalkoxy, substituted or unsubstituted lower alkenyl, substituted or unsubstituted cycloalkenyl, substituted or unsubstituted aryl, a substituted or unsubstituted aromatic heterocyclic group, substituted or unsubstituted aralkyl, or -CONR⁹R¹⁰ (wherein R⁹ and R¹⁰ may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R⁹ and R¹⁰ form a substituted or unsubstituted heterocyclic group together with an adjacent nitrogen atom), or two groups on the same carbon atom among R¹, R², R³ and R⁴ form a saturated carbon ring together with the carbon atom, two groups on two adjacent carbon atoms among R¹, R², R³ and R⁴ form a saturated carbon ring with said two adjacent carbon atoms, or two groups on two adjacent carbon atoms among R¹, R², R³ and R⁴ are combined to represent a bond (forming a double bond together with the already existing bond); R⁵ and R⁶ may be the same or different and each represents a hydrogen atom, hydroxy, halogen, substituted or unsubstituted lower alkoxy, substituted or unsubstituted lower alkanoyloxy, substituted or unsubstituted aralkyloxy, or substituted or unsubstituted aryloxy; R^{7a} represents hydroxy, formyl, substituted or unsubstituted lower alkoxy, -COQ (wherein Q represents halogen), -NR¹¹R¹² (wherein R¹¹ and R¹² may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R¹¹ and R¹² form a substituted or unsubstituted heterocyclic group together with an adjacent nitrogen atom), -COOR¹³ (wherein R¹³ represents a hydrogen atom, or substituted or unsubstituted lower alkyl),-CONR¹⁴R¹⁵ (wherein R¹⁴ and R¹⁵ may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R¹⁴ and R¹⁵ form a substituted or unsubstituted heterocyclic group together with an adjacent nitrogen atom), or -CH₂COOR¹⁶ (wherein R¹⁶ represents a hydrogen atom, or substituted or unsubstituted lower alkyl); and R⁸ represents a hydrogen atom, or substituted or unsubstituted lower alkoxy, or R^{7a} and R⁸ are combined together to represent -OCH₂(CH₂)ₚO- (wherein p represents an integer of 1 to 3), -CR¹⁷R¹⁸O- (wherein R¹⁷ and R¹⁸ may be the same or different and each represents a hydrogen atom or cyano), =CHOR¹⁹ (wherein R¹⁹ represents substituted or unsubstituted lower alkyl, substituted or unsubstituted lower alkenyl, or substituted or unsubstituted aralkyl), =CHCOOR²⁰ (wherein R²⁰ represents a hydrogen atom, or substituted or unsubstituted lower alkyl), or =O]

Compound (XIII) is disclosed in WO00/14085 as being useful as a PDE-IV inhibitor. A method for the preparation of a typical compound among compounds (XIII) disclosed in WO00/14085 is as follows:

### <Example 1 in WO00/14085>

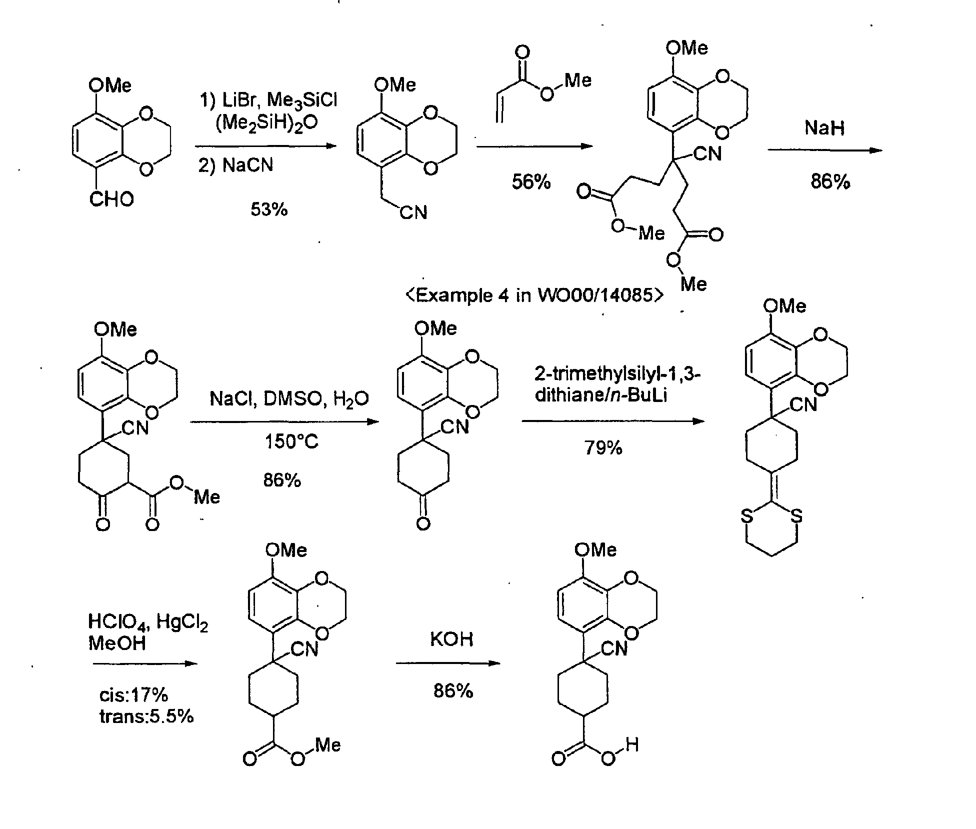

Me: Methyl, n-Bu: n-Butyl, DMSO: Dimethylsulfoxide

However, this method is not practically satisfactory as a industrially applicable preparation method, because of (1) requiring multiple steps, (2) low overall yield, (3) requiring purification by silica-gel column chromatography, and the like.

### Disclosure of the Invention

An object of the present invention is to provide compounds (I) that are useful as a synthetic intermediate for compound (XIII) or the like which is useful as a PDE-IV inhibitor, and effective methods for the preparation of the same.

Accordingly, the present invention provides the following aspects [1] to [8]:
[1] A compound represented by general formula (I): 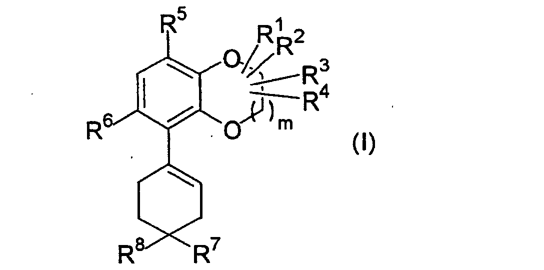 [wherein m represents an integer of 0 to 4;
   (i) R¹, R², R³ and R⁴ may be the same or different and each represents a hydrogen atom, substituted or unsubstituted lower alkyl, substituted or unsubstituted cycloalkyl, polycycloalkyl, substituted or unsubstituted lower alkoxycarbonyl, substituted or unsubstituted lower alkanoyl, substituted or unsubstituted lower alkanoyloxy, cyano, hydroxy, substituted or unsubstituted lower alkoxy, substituted or unsubstituted cycloalkoxy, substituted or unsubstituted lower alkenyl, substituted or unsubstituted cycloalkenyl, substituted or unsubstituted aryl, a substituted or unsubstituted aromatic heterocyclic group, substituted or unsubstituted aralkyl, or -CONR⁹R¹⁰ (wherein R⁹ and R¹⁰ may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R⁹ and R¹⁰ form a substituted or unsubstituted heterocyclic group together with an adjacent nitrogen atom);
   (ii) two groups on the same carbon atom among R¹, R², R³ and R⁴ form a saturated carbon ring together with the carbon atom;
   (iii) two groups'on two adjacent carbon atoms among R¹, R², R³ and R⁴ form a saturated carbon ring together with said two adjacent carbon atoms; or
   (iv) two groups on two adjacent carbon atoms among R¹, R², R³ and R⁴ are combined to represent a bond (forming a double bond together with the already existing bond); R⁵ and R⁶ may be the same or different and each represents a hydrogen atom, hydroxy, halogen, substituted or unsubstituted lower alkoxy, substituted or unsubstituted lower alkanoyloxy, substituted or unsubstituted aralkyloxy, or substituted or unsubstituted aryloxy;
      R⁷ represents hydroxy, formyl, cyano, substituted or unsubstituted lower alkoxy, -COQ (wherein Q represents halogen), -NR¹¹R¹² (wherein R¹¹ and R¹² may be the same or different and each represents a hydrogen atom, substituted or unsubstituted lower alkyl; substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R¹¹ and R¹² form a substituted or unsubstituted heterocyclic group together with an adjacent nitrogen atom), -COOR¹³ (wherein R¹³ represents a hydrogen atom, or substituted or unsubstituted lower alkyl),-CONR¹⁴R¹⁵ (wherein R¹⁴ and R¹⁵ may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R¹⁴ and R¹⁵ form a substituted or unsubstituted heterocyclic group together with an adjacent nitrogen atom) or -CH₂COOR¹⁶ (wherein R¹⁶ represents a hydrogen atom, or substituted or unsubstituted lower alkyl); and
      R⁸ represents a hydrogen atom, or substituted or unsubstituted lower alkoxy, or R⁷ and R⁸ are combined together to represent -OCH₂(CH₂)ₚO- (wherein p represents an integer of 1 to 3), -CR¹⁷R¹⁸O- (wherein R¹⁷ and R¹⁸ may be the same or different and each represents a hydrogen atom or cyano), =CHOR¹⁹ (wherein R¹⁹ represents substituted or unsubstituted lower alkyl, substituted or unsubstituted lower alkenyl, or substituted or unsubstituted aralkyl), =CHCOOR²⁰ (wherein R²⁰ represents a hydrogen atom, or substituted or unsubstituted lower alkyl), or =O], or a salt thereof.
[2] A process for preparing a compound represented by general formula (I): 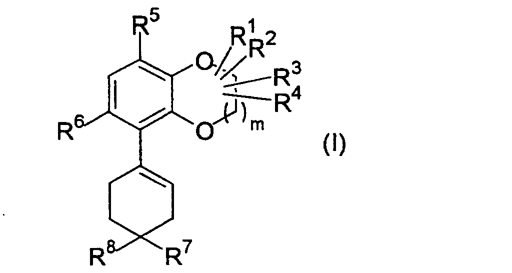 (wherein m, R¹_{,} R²_{,} R³_{,} R⁴, R⁵, R⁶, R⁷ and R⁸ have the same meanings as defined above, respectively), comprising:
   allowing a compound represented by general formula (II): 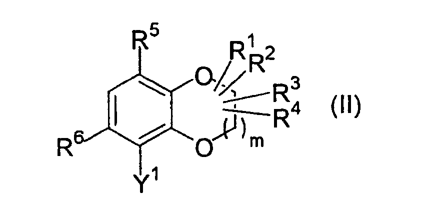 [wherein m, R¹, R², R³, R⁴, R⁵ and R⁶ have the same meanings as defined above, respectively, and Y¹ represents -B(OR²¹)₂ (wherein R²¹ represents a hydrogen atom, or substituted or unsubstituted lower alkyl)] to react with a compound represented by general formula (III): 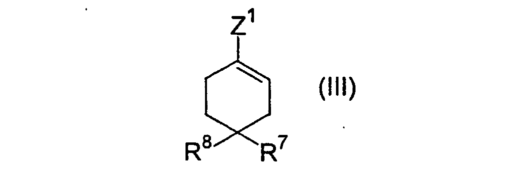 (wherein R⁷ and R⁸ have the same meanings as defined above, respectively, and Z¹ represents halogen or trifluoromethanesulfonate) in the presence of a palladium complex.
[3] A process for preparing a compound represented by general formula (I): 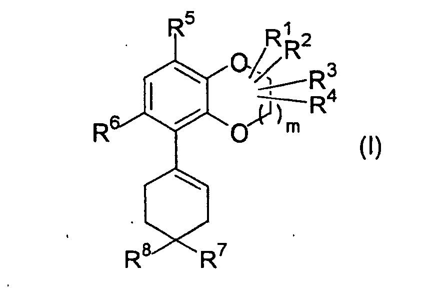 (wherein m, R¹, R², R³, R⁴, R⁵, R⁶, R⁷ and R⁸ have the same meanings as defined above, respectively), comprising:
   allowing a compound represented by general formula (IV): 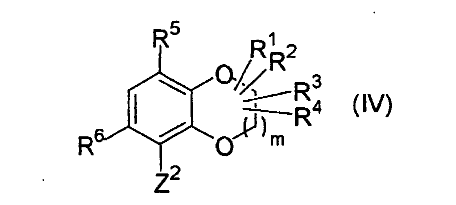 (wherein m, R¹, R², R³, R⁴, R⁵ and R⁶ have the same meanings as defined above, respectively, and Z² has the same meaning as Z¹ defined above) to react with a compound represented by general formula (V): 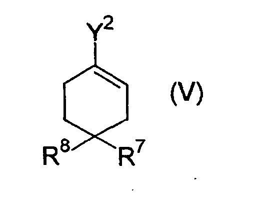 (wherein R⁷ and R⁸ have the same meanings as defined above, respectively, and Y² has the same meaning as Y¹ defined above) in the presence of a palladium complex.
[4] A process for preparing a compound represented by general formula (I): 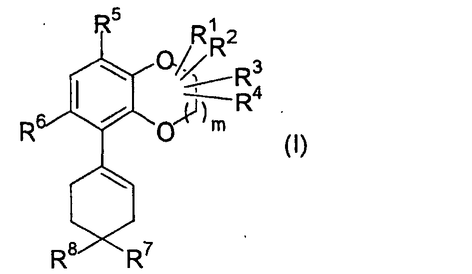 (wherein m, R¹, R², R³_{,} R⁴, R⁵, R⁶, R⁷ and R⁸ have the same meanings as defined above, respectively), comprising:
   allowing a compound represented by general formula (IX): 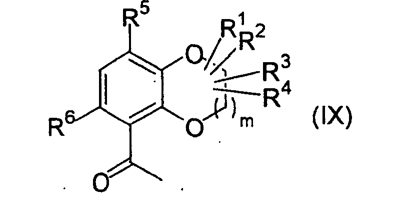 (wherein m, R¹, R², R³, R⁴, R⁵ and R⁶ have the same meanings as defined above, respectively) to react with a compound represented by general formula (X): 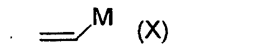 (wherein M represents a metal or a halogenated metal) to prepare a compound represented by general formula (VIII): 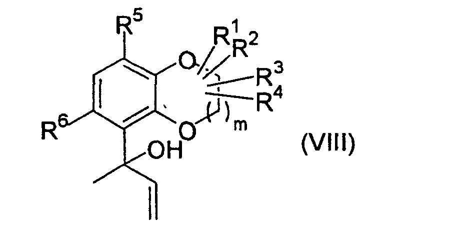 (wherein m, R¹, R², R³, R⁴, R⁵ and R⁶ have the same meanings as defined above, respectively); subsequently,
   converting the compound represented by general formula (VIII) into a compound represented by general formula (VI): 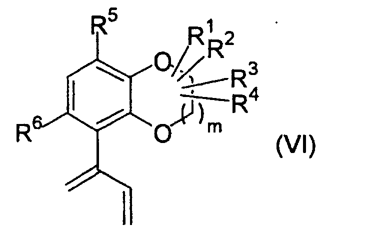 (wherein m, R¹, R², R³, R⁴, R⁵ and R⁶ have the same meanings as defined above, respectively) by dehydroation reaction; and then
   allowing the resulting compound to react with a compound represented by general formula (VII): 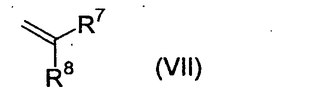 (wherein R⁷ and R⁸ have the same meanings as defined above, respectively) .
[5] A process for preparing a compound represented by general formula (I): 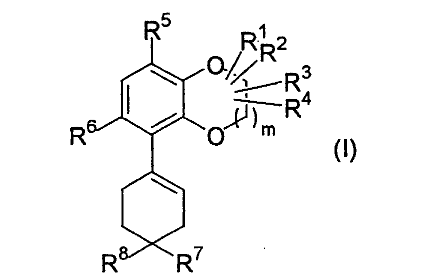 (wherein m, R¹, R², R³, R⁴, R⁵, R⁶, R⁷ and R⁸ have the same meanings as defined above, respectively), comprising:
   converting a compound represented by general formula (IV): 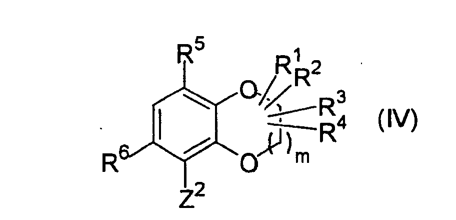 (wherein m, R¹, R², R³, R⁴, R⁵, R⁶ and Z² have the same meanings as defined above, respectively) into a corresponding organometallic compound wherein a metal atom is inserted between Z² and a benzene ring; subsequently,
   allowing the resulting compound to react with a compound represented by general formula (XI): 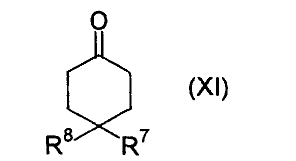 (wherein R⁷ and R⁸ have the same meanings as defined above, respectively) to give a compound represented by general formula (XII): 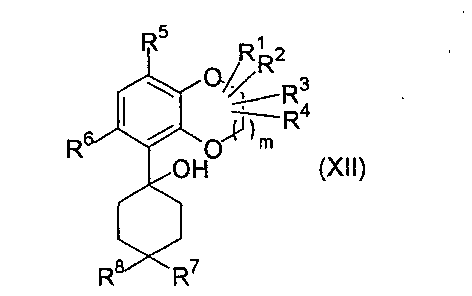 (wherein m, R¹, R², R³, R⁴, R⁵, R⁶, R⁷ and R⁸ have the same meanings as defined above, respectively); and then
   subjecting the resulting compound to dehydration reaction.
[6] The process according to the above described [5], wherein the corresponding organometallic compound wherein a metal atom is inserted between Z² and a benzene ring is an organotitanium compound.
[7] A compound represented by general formula (VI): 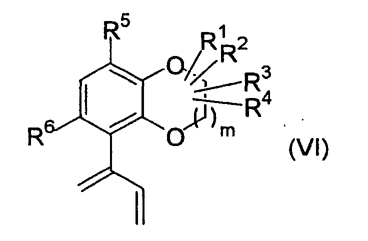 (wherein m, R¹, R², R³, R⁴, R⁵ and R⁶ have the same meanings as defined above, respectively), or a salt thereof.
[8] A compound represented by general formula (VIII): 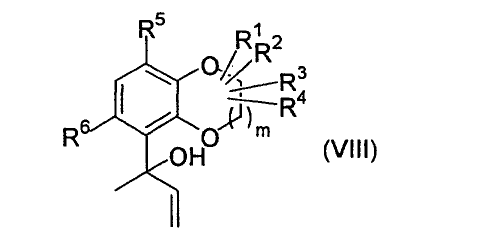 (wherein m; R¹, R², R³, R⁴, R⁵ and R⁶ have the same meanings as defined above, respectively), or a salt thereof.

In the definition of each group in the general formulas, examples of the lower alkyl and the lower alkyl moieties of the lower alkoxy and the lower alkoxycarbonyl include linear or branched alkyls having one to six carbon atom(s), such as methyl, ethyl, propyl, isopropyl, butyl, sec-butyl, tert-butyl, pentyl, isopentyl, neopentyl and hexyl.

Examples of the lower alkenyl include linear or branched alkenyls having two to six carbon atoms, such as vinyl, allyl, 1-propenyl, methacryl, crotyl, 1-butenyl, 3-butenyl, 2-pentenyl, 4-pentenyl, 2-hexenyl and 5-hexenyl.

Examples of the lower alkanoyl and the lower alkanoyl moiety of the lower alkanoyloxy include linear or branched alkanoyls having one to seven carbon atom(s), such as formyl, acetyl, propionyl, butyryl, isobutyryl, valeryl, isovaleryl, pivaloyl, hexanoyl and heptanoyl.

Examples of the cycloalkyl and the cycloalkyl moiety of the cycloalkoxy include cycloalkyls having three to eight carbon atoms, such as cyclopropyl, cyclobutyl, cyclopentyl, cyclohexyl, cycloheptyl and cyclooctyl.

Examples of the polycycloalkyl include polycycloalkyls having five to twelve carbon atoms, such as bicyclo[3.2.1]octyl, bicyclo[4.3.2]undecyl, adamantyl and noradamantyl.

Examples of the cycloalkenyl include cycloalkenyls having four to ten carbon atoms, such as cyclobutenyl, cyclopentenyl, cyclohexenyl, cycloheptenyl, cyclooctenyl, cyclononenyl and cyclodecenyl.

Examples of the aryl and the aryl moiety of the aryloxy include aryls having six to fourteen carbon atoms, such as phenyl, naphthyl and anthryl.

Examples of the aralkyl and the aralkyl moiety of the aralkyloxy include aralkyls having seven to fifteen carbon atoms, such as benzyl, phenethyl, benzhydryl and naphthylmethyl.

Examples of the aromatic heterocyclic group include five- or six- membered monocyclic aromatic heterocyclic groups containing at least one atom selected from a nitrogen atom, an oxygen atom and a sulfur atom, and bicyclic or tricyclic condensed aromatic heterocyclic groups comprising three- to eight-membered rings and containing at least one atom selected from a nitrogen atom, an oxygen atom and a sulfur atom, such as pyridyl, pyrazinyl, pyrimidinyl, pyridazinyl, quinolinyl, isoquinolinyl, phthalazinyl, quinazolinyl, quinoxalinyl, naphthyridinyl, cynnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl and purinyl.

Examples of the heterocyclic group formed together with an adjacent nitrogen atom include five-, six- or seven-membered monocyclic heterocyclic groups containing at least one nitrogen atom (said monocyclic heterocyclic groups may contain another nitrogen atom(s), oxygen atom(s) or sulfur atom(s)), and bicyclic or tricyclic condensed heterocyclic groups comprising three- to eight-membered rings and containing at least one nitrogen atom (said condensed heterocyclic groups may contain another nitrogen atom(s), oxygen atom(s) or sulfur atom(s)), such as pyrrolidinyl, piperidino, piperazinyl, morpholino, thiomorpholino, homopiperidino, homopiperazinyl, tetrahydropyridinyl, tetrahydroquinolinyl and tetrahydroisoquinolinyl.

Examples of the saturated carbon ring formed by two groups on the same carbon atom together with the carbon atom, and the saturated carbon ring formed by two groups on two adjacent carbon atoms together with said two carbon atoms include saturated carbon rings having three to ten carbon atoms, such as cyclopropane, cyclobutane, cyclopentane, cyclohexane, cycloheptane, cyclooctane, cyclononane and cyclodecane.

The halogen represents a fluorine, chlorine, bromine or iodine atom.

Examples of the metal include sodium, potassium, lithium, zinc, aluminum and magnesium.

The substituents in the substituted lower alkyl, the substituted lower alkoxy, the substituted lower alkoxycarbonyl, the substituted lower alkanoyl, the substituted lower alkanoyloxy, the substituted lower alkenyl, the substituted cycloalkyl, the substituted cycloalkenyl, the substituted aryl, the substituted aryloxy, the substituted aralkyl, the substituted aralkyloxy, the substituted aromatic heterocyclic group and the substituted heterocyclic group formed together with an adjacent nitrogen atom include may be the same or different and include, for example, one to five substituent(s), such as lower alkyl, cycloalkyl, lower alkoxycarbonyl, carboxy, amino, cyano, nitro, carbamoyl, trifluoromethyl, lower alkanoyl, lower alkanoyloxy, lower alkoxy, aralkyloxy, hydroxy, substituted or unsubstituted aryloxy, substituted or unsubstituted aryl, aralkyl and halogen. Herein, the substituents in the substituted aryl and the substituted aryloxy may be the same or different and include, for example, one to three substituent(s), such as lower alkyl and lower alkoxy, and the position(s) of the substituent(s) are not particularly limited. Herein, the lower alkyl, the cycloalkyl, the lower alkoxycarbonyl, the lower alkanoyl, the lower alkanoyloxy, the lower alkoxy, the aralkyloxy, the aryloxy, the aryl, the aralkyl and the halogen have the same meanings as defined above, respectively.

Examples of the salts of compounds (I), (VI) and (VIII) include acid addition salts, metal salts, ammonium salts, organic amine addition salts and amino acid addition salts, that are subjected to the reactions.

Examples of the acid addition salts subjected to the reactions for compounds (I), (VI) and (VIII) include inorganic acid salts, such as chlorides, sulfates, nitrates, phosphates and fluorosulfonates; and organic acid salts, such as acetate, maleates, fumarates, citrates, methanesulfonates, p-toluenesulfonates and trifluoromethanesulfonates. Examples of the metal salts subjected to the reactions include alkali metal salts such as sodium salts and potassium salts; alkaline earth metal salts such as magnesium salts and calcium slats; aluminum salts; and zinc salts. Examples of the ammonium salts subjected to the reactions include ammonium and tetramethylammonium. Examples of the organic amine addition salts subjected to the reactions include addition salts with morpholine or piperidine. Examples of the amino acid addition salts subjected to the reactions include addition salts with glycine, phenylalanine, lysine, aspartic acid or glutamic acid.

In the compounds of the present invention, preferably, R⁵ in the formula is lower alkoxy, R⁷ is -COOR¹³ (wherein R¹³ is as defined above), and more preferably, R⁵ is lower alkoxy, R⁶ is a hydrogen atom, R⁷ is -COOR¹³ (wherein R¹³ is as defined above), and R⁸ is a hydrogen atom.

In the process for the preparation according to the present invention, compound (I) can be prepared by the following steps.

### Method 1

Compound (I) can be prepared by the following step:

### Method 1

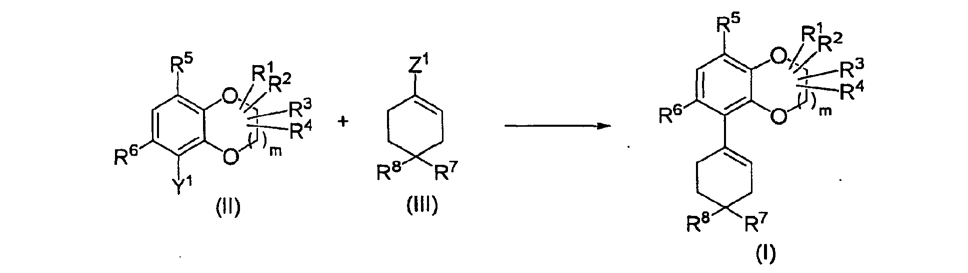

(wherein m, R¹, R², R³, R⁴, R⁵, R⁶, R⁷, R⁸, Y¹ and Z¹ have the same meanings as defined above, respectively.)

Compound (I) can be prepared by the reaction of compound (II) with one equivalent to an excess amount of compound (III) in the presence of catalytic to an excess amount of a palladium complex in the inert solvent, optionally, in the presence of a base and in the presence of a salt.

Examples of the palladium complex include tetrakis (triphenylphosphine)palladium, dichlorobis(triphenylphosphine)palladium, dichlorobis(acetonitrile)palladium, [1,1'-bis(diphenylphosphino)ferrocene]dichloropalladium, and combinations for forming the palladium complex in the reaction system such as a combination of palladium carbon and triphenylphosphine and a combination of palladium acetate and triphenylphosphine. In particular, the combination of the palladium carbon and triphenylphosphine is preferably used.

Examples of the base include inorganic bases such as potassium carbonate, sodium carbonate, cesium carbonate, potassium phosphate, sodium hydroxide and potassium hydroxide; and organic bases such as pyridine and triethylamine. In particular, carbonates such as potassium carbonate, sodium carbonate and cesium carbonate are preferably used.

Examples of the salt include cesium fluoride, lithium iodide and lithium bromide.

The palladium complex is used in an amount of preferably 0.0001 to 2 equivalents and more preferably 0.01 to 0.05 equivalents for compound (II).

The base is used in an amount of preferably 1 to 10 equivalents and more preferably 1 to 2 equivalents for compound (II).

The salt is used in an amount of preferably 1 to 10 equivalents and more preferably 1 to 2 equivalents for compound (II).

Examples of the inert solvent include tetrahydrofuran (THF), dioxane, diethyl ether, 1,2-dimethoxyethane (DME), diethylene glycol dimethyl ether, ethyl acetate, toluene, hexane, methanol, ethanol, chloroform, dimethylformamide (DMF), dimethyl sulfoxide (DMSO) and water. These may be used alone or in combination in an amount of preferably 1 to 100 times (by volume) compound (II).

The reaction is generally carried out at a temperature between 0°C and the boiling point of the used solvent, and will be completed within 5 minutes to 24 hours.

Compound (II) can be prepared according to the method described in WO00/14085 or modified method thereof and compound (III) can be prepared according to the method described in J. Am. Chem. Soc., 111, 8320(1989) or modified method thereof.

### Method 2

Compound (I) can also be prepared by the following step:

### Method 2

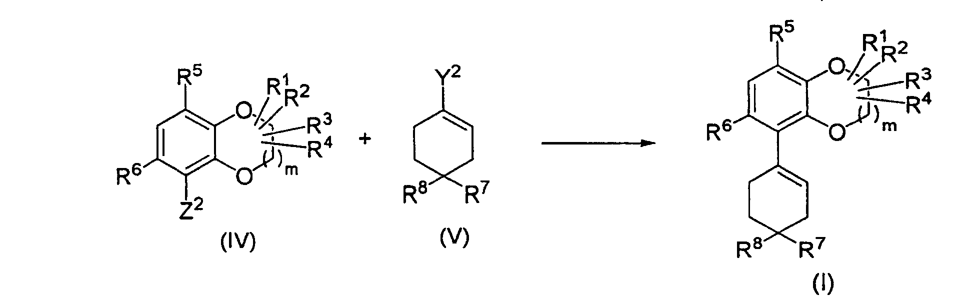

(wherein m, R¹, R², R³, R⁴, R⁵, R⁶, R⁷, R⁸, Y² and Z² have the same meanings as defined above, respectively.)

Compound (I) can be prepared by the reaction of compound (IV) with one equivalent to an excess amount of compound (V) in the presence of catalytic to an excess amount of palladium complex in an inert solvent, optionally, in the presence of a base and in the presence of a salt.

Examples of the palladium complex include tetrakis(triphenylphosphine)palladium, dichlorobis(triphenylphosphine)palladium, dichlorobis(acetonitrile)palladium, [1,1'-bis(diphenylphosphino)ferrocene]dichloropalladium and combinations for forming the palladium complex in the reaction system such as a combination of palladium carbon and triphenylphosphine, and a combination of palladium acetate and triphenylphosphine. In particular, the combination of palladium carbon and triphenylphosphine is preferably used.

Examples of the base include inorganic bases such as potassium carbonate, sodium carbonate, cesium carbonate, potassium phosphate, sodium hydroxide and potassium hydroxide; and organic bases such as pyridine and triethylamine. In particular, carbonates such as potassium carbonate, sodium carbonate and cesium carbonate are preferably used.

Examples of the salt include cesium fluoride, lithium iodide and lithium bromide.

The palladium complex is used in an amount of preferably 0.0001 to 2 equivalents and more preferably 0.01 to 0.05 equivalents for compound (IV).

The base is used in an amount of preferably 1 to 10 equivalents and more preferably 1 to 2 equivalents for compound (IV).

The salt is used in an amount of preferably 1 to 10 equivalents and more preferably 1 to 2 equivalents for compound (IV).

Examples of the inert solvent include THF, dioxane, diethyl ether, DME, diethylene glycol dimethyl ether, ethyl acetate, toluene, hexane, methanol, ethanol, chloroform, DMF and water. These may be used alone or in combination in an amount of preferably 1 to 100 times (by volume) compound (IV) .

The reaction is generally carried out at a temperature between 0°C and the boiling point of the used solvent, and will be completed within 5 minutes to 24 hours.

Compound (IV) can be prepared according to the method described in WO98/22455 or modified method thereof, and compound (V) can be prepared according to the method described in J. Org. Chem., 60, 7508(1995) or modified method thereof.

### Method 3

Compound (I) can also be prepared by the following steps A-1 to A-3:

### Method 3

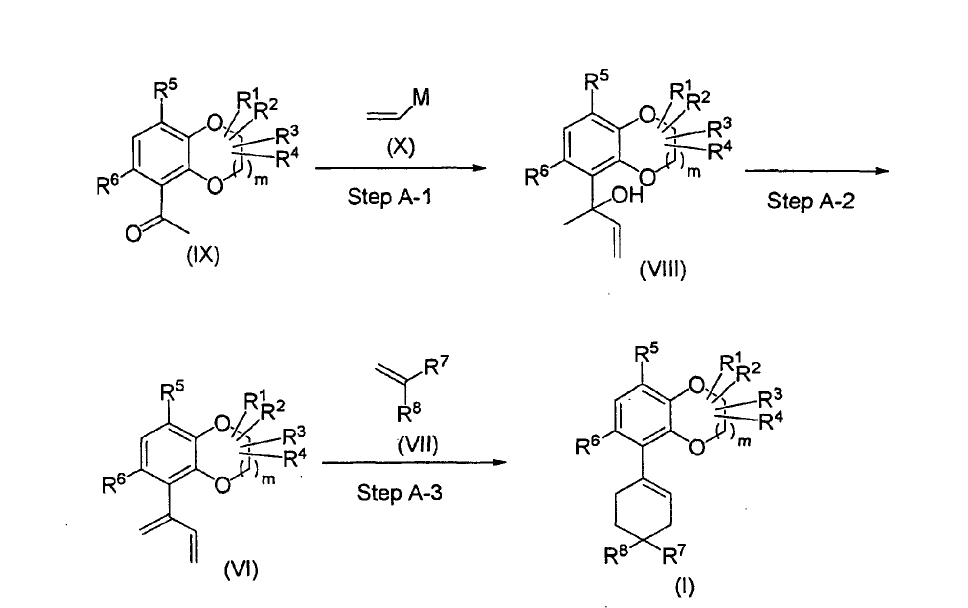

(wherein m, R¹, R², R³, R⁴, R⁵, R⁶, R⁷, R⁸ and M have the same meanings as defined above, respectively.)

In these steps, each intermediate can be used for the next step without isolation and purification. In particular, in the steps A-2 and A-3, the intermediate are preferably used without isolation. That is, compound (VIII) is converted into compound (VI) by dehydration, immediately compound (VI) is allowed to react with compound (VII) that is present in the reaction system.

### Step A-1

Compound (VIII) can be prepared by the reaction of compound (IX) with one to an excess amount of compound (X) in an inert solvent.

Examples of the inert solvent include THF, dioxane, diethyl ether, DME, diethylene glycol dimethyl ether, ethyl acetate, toluene, hexane and chloroform. These may be used alone or in combination in an amount of preferably 1 to 100 times (by volume) compound (IX).

The reaction is generally carried out at a temperature between -78°C and the boiling point of the used solvent, and will be completed within 5 minutes to 24 hours.

Compound (IX) can be prepared according to the method described in Chem. Ber., 105, 3301(1972) or modified method thereof, and compound (X) is commercially available.

### Step A-2

Compound (VI) can be prepared by the dehydration of compound (VIII) in an inert solvent, if necessary, in the presence of an acid catalyst.

Examples of the acid catalyst include pyridinium p-toluenesulfonate and p-toluenesulfonic acid, and the used amount of the acid catalyst is preferably in the range of 0.001 to 1 equivalent for compound (VIII).

Examples of the inert solvent include THF, dioxane, diethyl ether, DME, diethylene glycol dimethyl ether, ethyl acetate, n-propyl acetate, toluene, hexane, chloroform and benzotrifluoride. These may be used alone or in combination in an amount of preferably 1 to 100 times (by volume) compound (VIII).

The reaction is generally carried out at a temperature between 0°C and the boiling point of the used solvent, and will be completed within 5 minutes to 24 hours. The reaction is preferably carried out in the presence of the acid. When the acid is not used, the reaction is preferably carried out at a temperature between 50°C and the boiling point of the used solvent.

### Step A-3

Compound (I) can be prepared by the reaction of compound (VI) with compound (VII) in an inert solvent, if necessary, in the presence of a Lewis acid catalyst.

Examples of the Lewis acid catalyst include boron trifluoride etherate, zinc chloride, scandium triflate and dimethylaluminium chloride, and the amount of used thereof is preferably in the range of 0.01 to 5 equivalents for compound (VI).

Examples of the inert solvent include THF, dioxane, diethyl ether, DME, diethylene glycol dimethyl ether, ethyl acetate, toluene, hexane, chloroform, benzotrifluoride and acetone. These may be used alone or in combination in an amount of preferably 1 to 100 times (by volume) compound (VI).

The reaction is generally carried out at a temperature between 0°C and the boiling point of the used solvent, and will be completed within 5 minutes to 24 hours.

### Method 4

Compound (I) can also be prepared by the following steps B-1 to B-2:

### Method 4

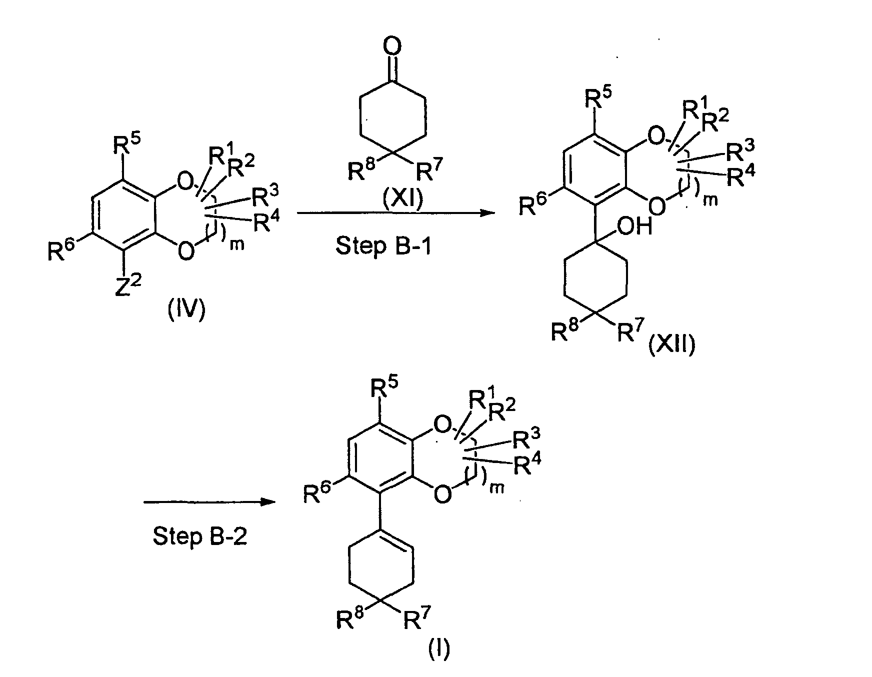

(wherein m, R¹, R², R³, R⁴, R⁵, R⁶_{,} R⁷_{,} R⁸ and Z² have the same meanings as defined above, respectively.)

### Step B-1

Compound (XII) can be prepared by treatment of compound (IV) with a base or the like to form the corresponding organometallic compound inserting a metal between Z² and the benzene ring, and following to react with one equivalent to an excess amount of compound (XI) in an inert solvent.

A Example of the method for preparation of the corresponding organic metallic compound inserting a metal between Z² and the benzene ring includes the treatment of compound (IV) with, for example, butyllithium and the like to form the lithio compound, followed by the treatment with chlorotitanium triisopropoxide to convert into an organotitanium compound. Another example of the method for the preparation includes the treatment of compound (IV) with, for example, butyllithium and the like to form a litio compound followed by the treatment with cerium chloride to convert into an organocerium compound.

Examples of the inert solvent include THF, dioxane, diethyl ether, DME, diethylene glycol dimethyl ether, toluene, hexane and chloroform. These may be used alone or in combination in an amount of preferably 1 to 100 times (by volume) compound (IV).

The reaction is generally carried out at a temperature between -100°C and the boiling point of the used solvent, and will be completed within 5 minutes to 24 hours.

### Step B-2

Compound (I) can be prepared by the dehydration of compound (XII) in an inert solvent, if necessary, in the presence of an acid.

Examples of the acid include trifluoromethanesulfonic acid, p-toluenesulfonic acid, methanesulfonic acid, hydrochloric acid, acetic acid, sulfuric acid and trifluoroacetic acid. Trifluoroacetic acid or hydrochloric acid is preferably used.

Examples of the inert solvent include THF, dioxane, diethyl ether, DME, diethylene glycol dimethyl ether, toluene, hexane, ethyl acetate and chloroform. These may be used alone or in combination in an amount of preferably 1 to 100 times (by volume) compound (XII).

The reaction is generally carried out at a temperature between 0°C and the boiling point of the used solvent, and will be completed within 5 minutes to 24 hours.

Furthermore, compound (XIV) described below including compound (XIII) can be produced by the following method described below.

### Method 5

Compound (XIV) can be prepared by the following step:

### Method 5

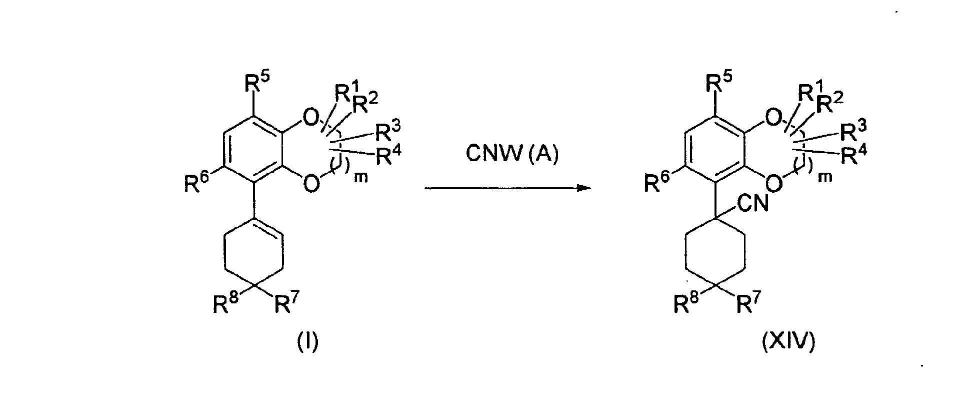

[wherein m, R¹, R², R³, R⁴, R⁵, R⁶, R⁷ and R⁸ have the same meanings as defined above, respectively; W represents a hydrogen atom, metal, substituted or unsubstituted lower alkyl, or -SiR²¹ R²²R²³ (wherein R²¹, R²² and R²³ may be the same or different and each represents a hydrogen atom or substituted or unsubstituted lower alkyl).]

Herein the substituted or unsubstituted lower alkyl is as defined above. Examples of metals include alkaline metals such as sodium, potassium and lithium, zinc and aluminum.

Compound (XIV) can be prepared by the reaction of compound (I) with compound (A) in the presence of a strong Bronsted acid in an inert solvent.

The strong Bronsted acid preferably has a pKa value of 3.0 or less, more preferably 1.0 or less, and most preferably 0.0001 to 1.0. Examples of the strong Bronsted acid include mineral acids, such as hydrochloric acid, hydrobromic acid, hydroiodic acid, perchloric acid, perbromic acid, periodic acid, sulfuric acid and phosphoric acid; carboxylic acids such as trifluoroacetic acid; sulfonic acids such as halogen-substituted or unsubstituted aliphatic sulfonic acids having 1 to 20 carbon atom(s) (e.g., methanesulfonic acid, trifluoromethanesulfonic acid and 10-camphorsulfonic acid), lower alkyl-substituted or unsubstituted aromatic sulfonic acid having 6 to 20 carbon atoms (e.g., benzenesulfonic acid and toluenesulfonic acid) and fluorosulfonic acid. In particular, perchloric acid, perbromic acid, periodic acid, methanesulfonic acid, trifluoromethanesulfonic acid, benzenesulfonic acid, toluenesulfonic acid and 10-camphorsulfonic acid are preferably used. Herein, the halogen and the lower alkyl have the same meanings as defined above.

Examples of the inert solvent include hydrocarbons and halogenated hydrocarbons, e.g., pentane, hexane, cyclohexane, benzene, toluene, xylene, methylene chloride, chloroform, 1,2-dichloroethane, 1,1,1-trichlroethane, 1,1,2-trichlroethene, perfluorocyclohexane, chlorobenzene, dichlorobenzene, fluorobenzene and benzotrifluoride.

Examples of compound (A) include hydrogen cyanide, acetonecyanhydrine and trimethylsilyl cyanide.

Compound (A) is preferably used in an amount of 1 to 100 equivalents, and more preferably 1 to 5 equivalents for compound (I).

The strong Bronsted acid is preferably used in an amount of 0.01 to 100 equivalents, and more preferably 0.1 to 5 equivalents for compound (I).

The reaction is generally carried out at a temperature between -78°C and the boiling point of the used solvent, and preferably -30°C and 30°C, and will be completed within 5 minutes to 24 hours.

When the resulting compound (XIV) has an ester group which is not necessary, compound (XIV) may be deesterified by treatment of compound (XIV) in an inert solvent with an aqueous alkaline solution at a temperature between 0°C and the boiling point of the used solvent for 5 minutes to 48 hours.

Examples of the aqueous alkaline solution include aqueous solutions of sodium hydroxide, potassium hydroxide and lithium hydroxide.

Examples of the inert solvent include ethanol, dioxane, methanol, THF and DMSO. A mixed solvent of ethanol and THF, and a mixed solvent of methanol and THF, and the like may also be used.

In addition to the above methods, the transformation of the functional groups contained in R¹, R², R³, R⁴, R⁵, R⁶, R⁷ and R⁸ in compounds (I) and (XIV) may be also performed by combining other known methods [for example, "Comprehensive Organic Transformations" by R. C. Larock (1989)].

The intermediates and the desired compounds in the above methods may be isolated and purified by separation and purification methods that are generally employed in organic synthetic chemistry, for example, filtration, extraction, washing, drying, concentration, recrystallization and various types of chromatography. The intermediates may be used for the next reactions without purification.

To obtain a salt of compound (I), (VI), or (VIII), when compound (I), (VI) or (VIII) prepared as a salt, it can be purified as it is. When compound (I), (VI) or (VIII) prepared as a free form, it may be dissolved or suspended into a suitable solvent and then may be isolated and purified by addition of an acid or a base. Compound (I), (VI) or (VIII) and the salt thereof may be present in the form of an adduct with water or another solvent in some cases. The adduct is also included in the present invention.

Compound (XIV) prepared by above methods is useful as, for example, a PDE-IV inhibitor (WO98/22455 or WO00/14085).

The present invention will now be described in further detail with by EXAMPLES and REFERENCE EXAMPLES. However, the present invention is not limited to EXAMPLES and REFERENCE EXAMPLES.

### Best Mode for Carrying Out the Invention

### EXAMPLE 1: Synthesis of 4-(2,3-dihydro-8-methoxy-1,4-benzodioxine-5-yl)-3-cyclohexenecarboxylic acid ethyl ester

To a mixture of 5% palladium carbon (50 mg), triphenylphosphine (42 mg), lithium bromide (260 mg), an 0.2 mol/L aqueous sodium carbonate (10 mL) and DME (10 ml) was added (2,3-dihydro-8-methoxy-1,4-benzodioxine-5-yl)boric acid (210 mg) prepared according to the method described in WO00/14085 or modified method thereof and a solution of ethyl 4-trifluoromethanesulfonyl-3-cyclohexenecarboxylate (363 mg) prepared according to the method described in J. Am. Chem. Soc., 111, 8320(1989) or modified method thereof in DME (10 ml). Under a nitrogen atmosphere, the mixture was heated under reflux for 1 hour. Ethyl acetate and brine were added to the reaction mixture. After palladium carbon was removed by filtration, the mixture was extracted. The organic layer was dried over anhydrous magnesium sulfate and was concentrated under reduced pressure. The residue was crystallized from ethanol (0.6 ml) to afford 4-(2,3-dihydro-8-methoxy-1,4-benzodioxine-5-yl)-3-cyclohexenecarboxylic acid ethyl ester (233 mg, 73.2%) as a solid.
Boiling point 88°C
¹H-NMR (CDCl₃, δ ppm) 6.65 (d, J = 8.5 Hz, 1H), 6.44 (d, J = 8.5 Hz, 1H), 5.80-5.75 (m, 1H), 4.32-4.24 (m, 4H), 4.16 (q, J = 7.2 Hz, 2H), 3.86 (s, 3H), 2.50-2.41 (m, 1H), 2.31-2.19 (m, 4H), 2.01-1.90 (m, 1H), 1.67-1.54 (m, 1H), 1.27 (t, J = 7.2 Hz, 3H).
IR (KBr, cm⁻¹) 2932, 1720, 1607, 1502, 1458, 1373, 1286, 1169, 1115.
MS (m/z) 319(M+H)⁺.

### EXAMPLE 2: Synthesis of 4-(2,3-dihydro-8-methoxy-1,4-benzodioxine-5-yl)-3-cyclohexenecarboxylic acid ethyl ester

### (1) Synthesis of 3-(2,3-dihydro-8-methoxy-1,4-benzodioxine-5-yl)-1-butene-3-ol

Under a nitrogen atmosphere, to a solution of 0.80 mol/L of vinylmagnesium bromide in THF (288 mL) was added THF (240 mL), and a solution of 1-(2,3-dihydro-8-methoxy-1,4-benzodioxine-5-yl)ethane-1-one (30.0 g) prepared according to the method described in Chem. Ber., 105, 3301(1972) in THF (120 mL) was added dropwise at 5°C. After being stirred for 45 minutes, an aqueous saturated ammonium chloride was added, and the mixture was extracted with ethyl acetate. The organic layer was washed with brine and dried over anhydrous sodium sulfate. The solvent was evaporated under reduced pressure to give 3-(2,3-dihydro-8-methoxy-1,4-benzodioxine-5-yl)-1-butene-3-ol (32.9 g, 96.5%) as a liquid. ¹H-NMR (CDCl₃, δ ppm) 6.83 (d, J = 8.7 Hz, 1H) , 6.47 (d, J = 8.7 Hz, 1H), 6.18 (dd, J = 17.2, 10.6 Hz, 1H), 5.11 (dd, J = 17.2, 1.1 Hz, 1H), 5.03 (dd, J = 10.6, 1.1 Hz, 1H), 4.36-4.25 (m, 4H), 3.87 (s, 3H), 1.64 (s, 3H).
MS (m/z) 235(M-H)⁻.

### (2) Synthesis of 4-(2,3-dihydro-8-methoxy-1,4-benzodioxine-5-yl)-3-cyclohexenecarboxylic acid ethyl ester

To a solution of 3-(2,3-dihydro-8-methoxy-1,4-benzodioxine-5-yl)-1-butene-3-ol (32.8 g) prepared in (1) of EXAMPLE 2 in n-propyl acetate (164 mL) was added Ethyl acrylate (75.6 mL) and pyridinium p-toluenesulfonate (105 mg), and the mixture was heated under reflux for 9 hours under a nitrogen atmosphere. An aqueous saturated sodium hydrogen carbonate and brine were added to the mixture, the mixture was extracted. The solvent was evaporated under reduced pressure. The residue was crystallized from ethanol (34 mL) and water (6 mL) to give 4-(2,3-dihydro-8-methoxy-1, 4-benzodioxine-5-yl)-3-cyclohexenecarboxylic acid ethyl ester (31.2 g, 70.6%) as a solid.

### EXAMPLE 3: Synthesis of 4-(2,3-dihydro-8-methoxy-1,4-benzodioxine-5-yl)-3-cyclohexenecarboxylic acid ethyl ester

To a solution of 5-bromo-2,3-dihydro-8-methoxy-1,4-benzodioxine (94.3 g) prepared according to the method described in WO98/22455A in THF (820 mL) was added dropwise a solution of n-butyllithium in hexane (1.6 mol/L, 249 mL) at -60°C under a nitrogen atmosphere, and about 30 minutes later, a solution of chlorotitanium triisopropoxide (150.4 g) in THF (150 mL) was added dropwise. After being stired for 1 hour at 0°C, 4-oxocyclohexanecarboxylic acid ethyl ester (52.1 mL) was added, and the mixture was stirred for 4 hours at 15°C. Hydrochloric acid was added to the reaction mixture and the mixture was extracted. The organic layer was washed with brine and dried over anhydrous sodium sulfate. After the solvent was evaporated under reduced pressure, the residue was dissolved in acetonitrile (1,050 mL). Subsequently, trifluoroacetic acid (24.0 mL) was added and the mixture was stirred for 4 hours at room temperature. Ethyl acetate and an aqueous sodium hydrogen carbonate were added to the reaction mixture and the mixture was extracted, and the organic layer was dried over anhydrous sodium sulfate. After the solvent was evaporated under reduced pressure, the residue was crystallized from ethanol (210 mL) to give 4-(2,3-dihydro-8-methoxy-1,4-benzodioxine-5-yl)-3-cyclohexenecarboxylic acid ethyl ester (88.6 g, 72.3%) as a solid.

### EXAMPLE 4: Synthesis of 4-(2,3-dihydro-8-methoxy-1,4-benzodioxine-5-yl)-3-cyclohexenecarboxylic acid ethyl ester

To a solution of 3-(2,3-dihydro-8-methoxy-1,4-benzodioxine-5-yl)-1-butene-3-ol (42.3 g) prepared according to (1) of EXAMPLE 2 in toluene (423 mL) was added pyridinium p-toluenesulfonate (450 mg), and the mixture was heated under reflux for 1 hour, subsequently, water was removed with a Dean-Stark trap. The resulting solution was added dropwise to a mixture of acrylyl chloride (57.9 mL) and N,N-dimethylaniline (57.0 mL) at 10°C or below for 30 minutes, and the mixture was stirred for 3 hours in ice bath and followed by 3 hours at room temperature. Ethanol (83.5 mL) was added to the reaction mixture and the mixture was stirred for 10 minutes. After 1 mol/L hydrochloric acid (420 mL) was added, the mixture was extracted. The organic layer was washed with brine and the solvent was evaporated under reduced pressure. The reside was crystallized from ethanol (127 mL) to give 4-(2,3-dihydro-8-methoxy-1,4-benzodioxine-5-yl)-3-cyclohexenecarboxylic acid ethyl ester (40.0 g, 66.6%) as a solid.

### EXAMPLE 5: Synthesis of 4-(2,3-dihydro-8-methoxy-1,4-benzodioxine-5-yl)-3-cyclohexenecarboxylic acid ethyl ester

To a solution of 3-(2,3-dihydro-8-methoxy-1,4-benzodioxine-5-yl)-1-butene-3-ol (1.0 g) prepared in (1) of EXAMPLE 2 in toluene (20 mL) was added pyridinium p-toluenesulfonate (10.7 mg), and the mixture was heated under reflux for 1 hour, subsequently, water was removed with a Dean-Stark trap. To this solution was added a suspension of ethyl acrylate (4.6 mL) and zinc chloride (577 mg) in acetone, and the mixture was stirred for 10 hours at 50°C. Water was added to the reaction mixture, and the mixture was extracted. The organic layer was washed with brine and the solvent was evaporated under reduced pressure. The reside was crystallized from a mixed solvent of ethanol (2.55 mL) and water (0.45 mL) to give 4-(2,3-dihydro-8-methoxy-1,4-benzodioxine-5-yl)-3-cyclohexenecarboxylic acid ethyl ester (0.926 g, 66.5%) as a solid.

### EXAMPLE 6: Synthesis of 4-(2,3-dihydro-8-methoxy-1, 4-benzodioxine-5-yl)-3-cyclohexenecarbonitrile

To a solution of 3-(2,3-dihydro-8-methoxy-l,4-benzodioxine-5-yl)-1-butene-3-ol (0.5 g) prepared in (1) of EXAMPLE 2 in toluene (15 mL) was added pyridinium p-toluenesulfonate (5.0 mg), and the mixture was heated under reflux for 1 hour, subsequently, water was removed with a Dean-Stark trap. To this solution was added Acrylonitrile (1.4 mL), and the mixture was stirred for 20 hours at 70°C. Ethyl acetate and an aqueous saturated sodium hydrogen carbonate were added to the reaction mixture and the mixture was extracted. The organic layer was washed with brine and the solvent was evaporated under reduced pressure. The reside was purified by silica gel column chromatography (eluent: ethyl acetate/hexane=1/5 to 1/4) to give 4-(2,3-dihydro-8-methoxy-1,4-benzodioxine-5-yl)-3-cyclohexenecarbonitrile (0.517 g, 90.0%) as a solid.
Melting point 79°C
¹H-NMR (CDCl₃, δ ppm) 6.64 (d, J = 8.4 Hz, 1H), 6.43 (d, J= 8.4 Hz, 1H), 5.72-5.70 (m, 1H), 4.33-4.25 (m, 4H), 3.87 (s, 3H), 2.90-2.85 (m, 1H), 2.57-2.47 (m, 4H), 2.10-2.05 (m, 1H), 2.04-1.97 (m, 1H).
IR (KBr, cm⁻¹) 2932, 2233, 1605, 1501, 1443, 1285, 1173, 1119, 1049, 891, 787.
MS (m/z) 272(M+H)⁺.

### EXAMPLE 7: Synthesis of 4-(2,3-dihydro-8-methoxy-1,4-benzodioxine-5-yl)-3-cyclohexenecarboxylic acid ethyl ester

### (1) Synthesis of 4-(2,3-dihydro-8-methoxy-1,4-benzodioxine-5-yl)-3-cyclohexenecarboxylic acid

To 4-(2,3-dihydro-8-methoxy-1,4-benzodioxine-5-yl)-3-cyclohexenecarbonitrile (0.0366 g) prepared in EXAMPLE 6 was added an 10 mol/L aqueous sodium hydroxide (0.183 mL), water (0.183 mL) and ethanol (0.366 mL), and the mixture was heated under reflux for two days. The reaction mixture was acidified with 1 mol/L hydrochloric acid to give 4-(2,3-dihydro-8-methoxy-1,4-benzodioxine-5-yl)-3-cyclohexenecarboxylic acid (0.0286 g, 73.0%) as a solid. Melting point 186°C
¹H-NMR (DMSO-d₆, δ ppm) 12.13 (brs, 1H), 6.57 (d, J = 8.5 Hz, 1H), 6.47 (d, J = 8.5 Hz, 1H), 5.98 (brs, 1H) , 4.20-4.10 (m, 4H) , 3.70 (s, 3H), 2.67-2.57 (m, 1H), 2.48-2.38 (m, 4H), 2.14-2.06 (m, 1H), 1.87-1.74 (m, 1H).
IR (KBr, cm⁻¹) 3443, 2837, 1697, 1601, 1501, 1462, 1437, 1286, 1130, 1051, 951, 783. MS (m/z) 289(M-H)⁻.

### (2) Synthesis of 4-(2,3-dihydro-8-methoxy-1,4-benzodioxine-5-yl)-3-cyclohexenecarboxylic acid ethyl ester

To a solution of 4-(2,3-dihydro-8-methoxy-1,4-benzodioxine-5-yl)-3-cylcohexenecarboxlyic acid (0.0286 g) prepared in (1) of EXAMPLE 7 in ethanol (0.5 mL) was added sulfuric acid (40 mg), and the mixture was heated under reflux for 3 hours under a nitrogen atmosphere. Ethyl acetate and water were added to the reaction mixture and the mixture was extracted. The organic layer was washed with an aqueous sodium hydrogen carbonate and the solvent was evaporated under reduced pressure to give 4-(2,3-dihydro-8-methoxy-1,4-benzodioxine-5-yl)-3-cyclohexenecarboxylic acid ethyl ester (0.0271 g, 86.4%) as a solid.

### REFERENCE EXAMPLE 1: Synthesis of cis-4-cyano-4-(2,3-dihydro-8-methoxy-1,4-benzodioxine-5-yl)cyclohexanecarboxylic acid

### (1) Synthesis of cis-4-cyano-4-(2,3-dihydro-8-methoxy-1,4-benzodioxine-5-yl)cyclohexanecarboxylic acid ethyl ester

Under a nitrogen atmosphere, trifluoromethanesulfonic acid (2.25 g) and trimethylsilylcyanide (1.57 mL) were dissolved in benzotrifluoride (10 mL), and a solution of 4-(2,3-dihydro-8-methoxy-1, 4-benzodioxine-5-yl)-3-cyclohexenecarboxylic acid ethyl ester (0.79 g) prepared according to the method described in EXAMPLE 1 in benzotrifluoride (10 mL) was added dropwise at -25°C. After being stirred for for one hour at -20°C, an aqueous saturated sodium hydrogen carbonate was added and the mixture was extracted with ethyl acetate. The organic layer was washed with brine and dried over anhydrous magnesium sulfate, and the solvent was evaporated under reduced pressure. The residue was crystallized from ethanol (1 mL) to give a solid substance (0.64 g). The solid substance (0.030 g) was crystallized from a mixed solvent of diisopropyl ether and ethyl acetate (0.36 mL, diisopropyl ether/ethyl acetate = 4/1) to give cis-4-cyano-4-(2,3-dihydro-8-methoxy-1, 4-benzodioxine-5-yl)cyclohexanecarboxylic acid ethyl ester (0.019 g, 47.3%) as a solid.
Melting point 131°C
¹H-NMR (CDCl₃, δ ppm) 6.84 (d, J = 8.9 Hz, 1H), 6.49 (d, J = 8.9 Hz, 1H), 4.39-4.33 (m, 4H), 4.17 (q, J = 7.1 Hz, 2H), 3.88 (s, 3H), 2.44 (brd, J = 12.6 Hz, 2H), 2.32 (tt, J = 11.8, 3.8 Hz, 1H), 2.18-1.95 (m, 4H), 1.86 (dt, J = 3.6, 12.6 Hz, 2H), 1.28 (t, J = 7.1 Hz, 3H).
IR (KBr, cm⁻¹) 2953, 2228, 1722, 1607, 1504, 1460, 1381, 1325, 1281, 1117; 1043, 953, 787. MS (m/z) 346(M+H)⁺.

### (2) Synthesis of cis-4-cyano-4-(2,3-dihydro-8-methoxy-1,4-benzodioxine-5-yl)cyclohexanecarboxylic acid

To a suspension of cis-4-cyano-4-(2,3-dihydro-8-methoxy-1,4-benzodioxine-5-yl)cyclohexanecarboxylic acid ethyl ester (397 g) prepared according to the method described (1) of REFERENCE EXAMPLE 1 in ethanol (1.99 L) was added a 6 ml/L aqueous potassium hydroxide (377 mL), and the mixture was stirred for 4 hours at room temperature. After water (2.03 L) was added to the reaction mixture, a 6 mol/L aqueous hydrochloric acid (576 mL) was added to crystallize and to give cis-4-cyano-4-(2,3-dihydro-8-methoxy-1, 4-benzodioxine-5-yl)cyclohexanecarboxylic acid (366 g, 98.1%) as a solid.
Melting point 245°C
¹H-NMR (DMSO-d₆, δ ppm) 12.26 (brs, 1H), 6.79 (d, J = 8.9 Hz, 1H), 6.59 (d, J = 8.9 Hz, 1H), 4.27 (dd, J = 11.9, 5.0 Hz, 4H), 3.75 (s, 3H), 2.34-2.26 (m, 3H), 2.05-2.00 (m, 2H), 1.86-1.63 (m, 4H).
IR (KBr, cm⁻¹) 3287, 2932, 1728, 1609, 1508, 1454, 1285, 1119, 953, 802, 764.
MS (m/z) 318 (M+H) ⁺.

### Industrial Applicability

The present invention provides compound (I) that is useful as a synthetic intermediate of compound (XIII) and the like which are useful as a PDE-IV inhibitor, and the effective methods for the preparation of the same.

## Claims

1. A compound represented by general formula (I): 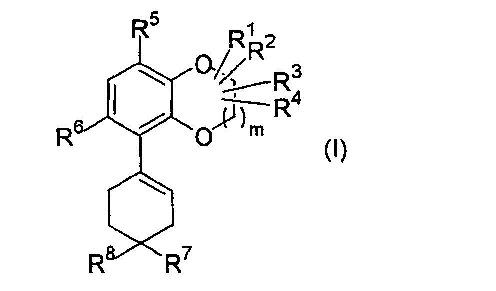 [wherein m represents an integer of 0 to 4;
(i) R¹, R², R³ and R⁴ may be the same or different and each represents a hydrogen atom, substituted or unsubstituted lower alkyl, substituted or unsubstituted cycloalkyl, polycycloalkyl, substituted or unsubstituted lower alkoxycarbonyl, substituted or unsubstituted lower alkanoyl, substituted or unsubstituted lower alkanoyloxy, cyano, hydroxy, substituted or unsubstituted lower alkoxy, substituted or unsubstituted cycloalkoxy, substituted or unsubstituted lower alkenyl, substituted or unsubstituted cycloalkenyl, substituted or unsubstituted aryl, a substituted or unsubstituted aromatic heterocyclic group, substituted or unsubstituted aralkyl, or -CONR⁹R¹⁰ (wherein R⁹ and R¹⁰ may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R⁹ and R¹⁰ form a substituted or unsubstituted heterocyclic group together with an adjacent nitrogen atom);
(ii) two groups on the same carbon atom among R¹, R², R³ and R⁴ form a saturated carbon ring together with the carbon atom;
(iii) two groups on two adjacent carbon atoms among R¹, R², R³ and R⁴ form a saturated carbon ring together with said two adjacent carbon atoms; or
(iv) two groups on two adjacent carbon atoms among R¹, R², R³ and R⁴ are combined to represent a bond (forming a double bond together with the already existing bond);
R⁵ and R⁶ may be the same or different and each represents a hydrogen atom, hydroxy, halogen, substituted or unsubstituted lower alkoxy, substituted or unsubstituted lower alkanoyloxy, substituted or unsubstituted aralkyloxy, or substituted or unsubstituted aryloxy;
R⁷ represents hydroxy, formyl, cyano, substituted or unsubstituted lower alkoxy, -COQ (wherein Q represents halogen), -NR¹¹R¹² (wherein R¹¹ and R¹² may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R¹¹ and R¹² form a substituted or unsubstituted heterocyclic group together with an adjacent nitrogen atom), -COOR¹³ (wherein R¹³ represents a hydrogen atom, or substituted or unsubstituted lower alkyl), C0NR¹⁴R¹⁵ (wherein R¹⁴ and R¹⁵ may be the same or different and each represents a hydrogen atom, substituted or unsubstituted lower alkyl, substituted or unsubstituted lower alkanoyl, substituted or unsubstituted cycloalkyl, substituted or unsubstituted aryl, a substituted or unsubstituted aromatic heterocyclic group, or substituted or unsubstituted aralkyl, or R¹⁴ and R¹⁵ form a substituted or unsubstituted heterocyclic group together with an adjacent nitrogen atom) or -CH₂COOR¹⁶ (wherein R¹⁶ represents a hydrogen atom, or substituted or unsubstituted lower alkyl); and
R⁸ represents a hydrogen atom, or substituted or unsubstituted lower alkoxy, or R⁷ and R⁸ are combined together to represent -OCH₂(CH₂)ₚO- (wherein p represents an integer of 1 to 3), -CR¹⁷R¹⁸O- (wherein R¹⁷ and R¹⁸ may be the same or different and each represents a hydrogen atom or cyano), =CHOR¹⁹ (wherein R¹⁹ represents substituted or unsubstituted lower alkyl, substituted or unsubstituted lower alkenyl, or substituted or unsubstituted aralkyl), =CHCOOR²⁰ (wherein R²⁰ represents a hydrogen atom, or substituted or unsubstituted lower alkyl), or =O], or a salt thereof.

2. A process for preparing a compound represented by general formula (I): 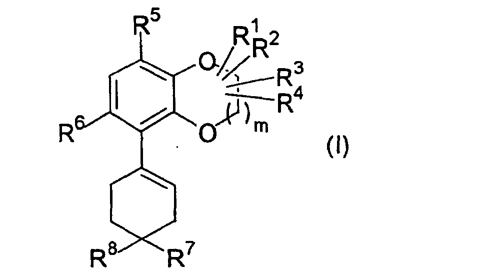 (wherein m, R¹, R², R³, R⁴, R⁵, R⁶, R⁷ and R⁸ have the same meanings as defined above, respectively), comprising:
allowing a compound represented by general formula (II) : 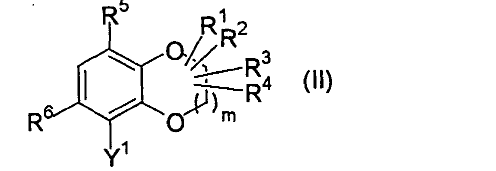 [wherein m, R¹, R², R³, R⁴, R⁵ and R⁶ have the same meanings as defined above, respectively, and Y¹ represents -B(OR²¹)₂ (wherein R²¹ represents a hydrogen atom, or substituted or unsubstituted lower alkyl)] to react with a compound represented by general formula (III): 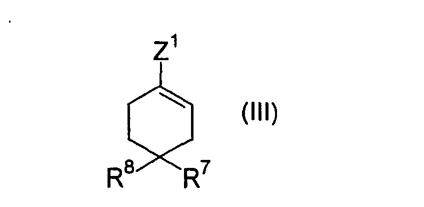 (wherein R⁷ and R⁸ have the same meanings as defined above, respectively, and Z¹ represents halogen or trifluoromethanesulfonate) in the presence of a palladium complex.

3. A process for preparing a compound represented by general formula (I): 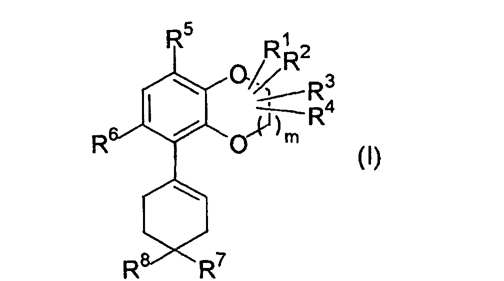 (wherein m, R¹, R², R³, R⁴, R⁵, R⁶, R⁷ and R⁸ have the same meanings as defined above, respectively), comprising:
allowing a compound represented by general formula (IV): 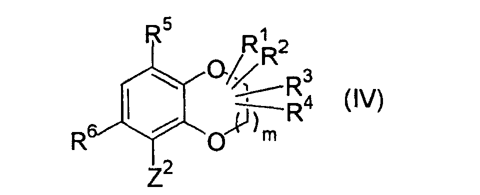 (wherein m, R¹, R², R³, R⁴, R⁵ and R⁶ have the same meanings as defined above, respectively, and Z² has the same meaning as Z¹ defined above) to react with a compound represented by general formula (V) : 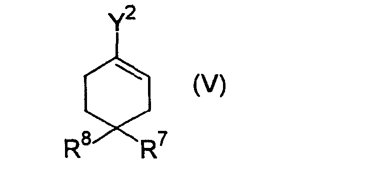 (wherein R⁷ and R⁸ have the same meanings as defined above, respectively, and Y² has the same meaning as Y¹ defined above) in the presence of a palladium complex.

4. A process for preparing a compound represented by general formula (I): 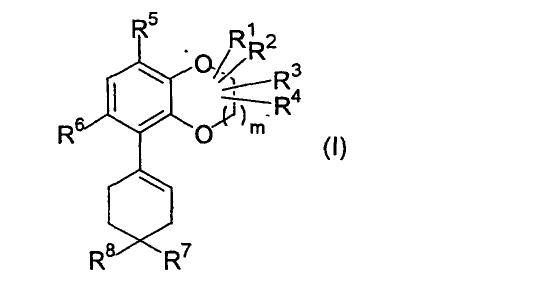 (wherein m, R¹, R², R³, R⁴, R⁵, R⁶, R⁷ and R⁸ have the same meanings as defined above, respectively), comprising:
allowing a compound represented by general formula (IX): 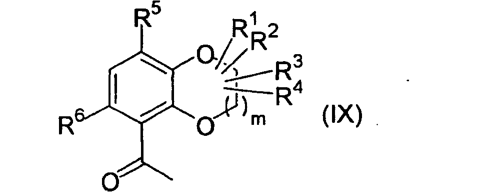 (wherein m, R¹, R², R³, R⁴, R⁵ and R⁶ have the same meanings as defined above, respectively) to react with a compound represented by general formula (X): 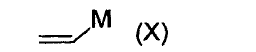 (wherein M represents a metal or a halogenated metal) to prepare a compound represented by general formula (VIII): 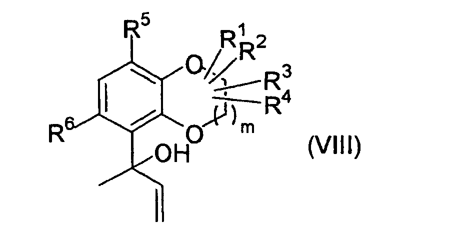 (wherein m, R¹, R², R³, R⁴, R⁵ and R⁶ have the same meanings as defined above, respectively); subsequently,
converting the compound represented by general formula (VIII) into a compound represented by general formula (VI): 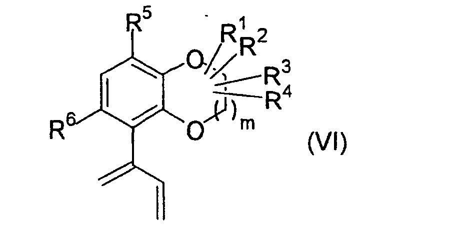 (wherein m, R¹, R², R³, R⁴, R⁵ and R⁶ have the same meanings as defined above, respectively) by dehydration reaction; and then
allowing the resulting compound (VI) to react with a compound represented by general formula (VII): 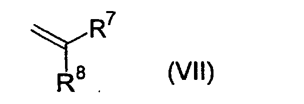 (wherein R⁷ and R⁸ have the same meanings as defined above, respectively).

5. A process for preparing a compound represented by general formula (I): 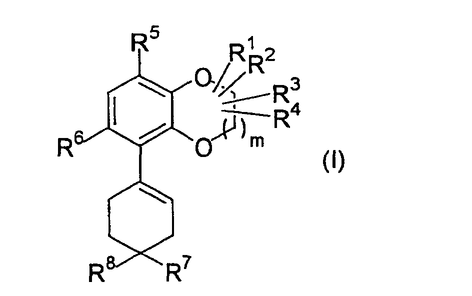 (wherein m, R¹, R², R³, R⁴, R⁵, R⁶, R⁷ and R⁸ have the same meanings as defined above, respectively), comprising:
converting a compound represented by general formula (IV): 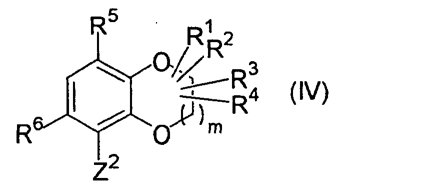 (wherein m, R¹, R², R³, R⁴, R⁵, R⁶ and Z² have the same meanings as defined above, respectively) into a corresponding organometallic compound wherein a metal atom is inserted between Z² and a benzene ring; subsequently,
allowing the resulting compound to react with a compound represented by general formula (XI): 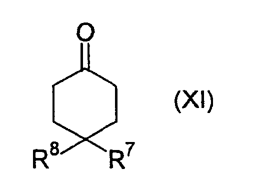 (wherein R⁷ and R⁸ have the same meanings as defined above, respectively) to give a compound represented by general formula (XII): 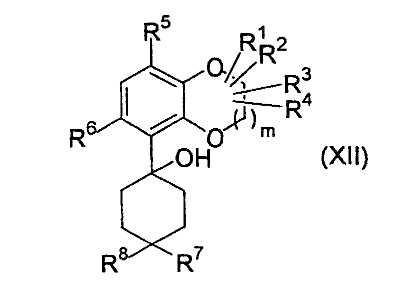 (wherein m, R¹, R², R³, R⁴, R⁵, R⁶, R⁷ and R⁸ have the same meanings as defined above, respectively); and then
subjecting the resulting compound to dehydration reaction.

6. The process according to claim 5, wherein the corresponding organometallic compound wherein a metal atom is inserted between Z² and a benzene ring is an organotitanium compound.

7. A compound represented by general formula (VI): 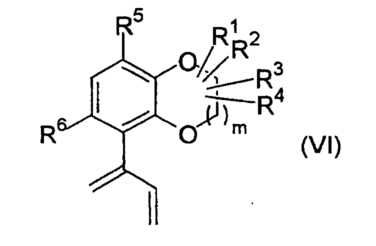 (wherein m, R¹, R², R³, R⁴, R⁵ and R⁶ have the same meanings as defined above, respectively), or a salt thereof.

8. A compound represented by general formula (VIII): 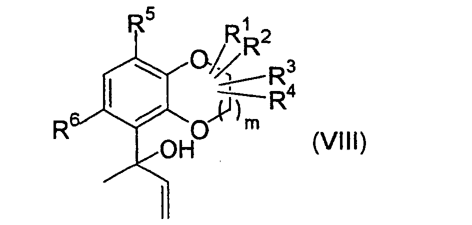 (wherein m, R¹, R², R³, R⁴, R⁵ and R⁶ have the same meanings as defined above, respectively), or a salt thereof.
